(19) Europäisches Patentamt
European Patent Office
Office européen des brevets

(11) Publication number: **0 078 142 B1**

# (12) EUROPEAN PATENT SPECIFICATION

(45) Date of publication of patent specification: **05.03.86**

(51) Int. Cl.[4]: **C 07 D 487/04,** C 08 K 5/34 // (C07D487/04, 235:00, 235:00)

(21) Application number: **82305548.8**

(22) Date of filing: **19.10.82**

## (54) Glycoluril derivatives as antioxidants, and processes for their production.

(30) Priority: **19.10.81 JP 167434/81**

(43) Date of publication of application: **04.05.83 Bulletin 83/18**

(45) Publication of the grant of the patent: **05.03.86 Bulletin 86/10**

(84) Designated Contracting States: **CH DE FR GB IT LI NL**

(56) References cited: **GB-A-2 010 875**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Terada, Yutaka**
**No. 8-12, Fukatani-cho Nishinomiya-shi**
**Hyogo (JP)**
Inventor: **Takahashi, Yokoh**
**No. 11-8-208 Sonehigashi-machi 2-chome**
**Toyonaka-shi Osaka (JP)**
Inventor: **Yachigo, Shinichi**
**No. 11-7-305 Sonehigashi-machi 2-chome**
**Toyonaka-shi Osaka (JP)**
Inventor: **Ishii, Tamaki**
**No. 12-2, Uchihonmachi 1-chome Suita-shi**
**Osaka (JP)**
Inventor: **Mochizuki, Masaharu**
**No. 1-9-205, Shirakawa 3-chome Ibaragi-shi**
**Osaka (JP)**

(74) Representative: **Diamond, Bryan Clive et al**
**Gee & Co. Chancery House Chancery Lane**
**London WC2A 1QU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 078 142 B1

Courier Press, Leamington Spa, England.

**Description**

The present invention relates to acetylene carbamide derivatives of the general formula (I):

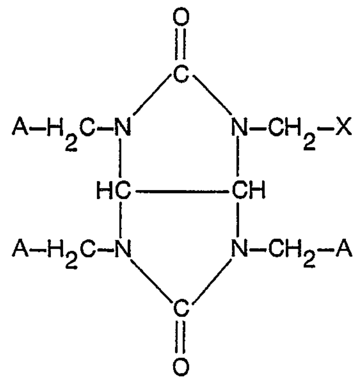

(I)

wherein A represents

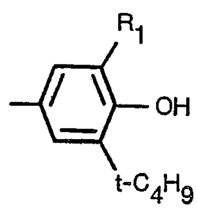

in which $R_1$ represents a hydrogen atom or an alkyl group having 1 to 4 carbon atoms, and X represents A or a hydrogen atom; processes for their production; and antioxidants for organic substances such as polymers which contain such derivatives as active ingredients.

Light or oxygen causes deterioration of various organic substances, including: synthetic resins, natural or synthetic rubbers, isoprene rubber, isoprene-isobutylene copolymer rubbers, styrene-butadiene copolymer rubbers, acrylonitrile-butadiene copolymer rubbers and ethylenepropylene-diene; petroleum products and various other organic substances such as fats, oils and greases. In order to inhibit such deterioration, various deterioration inhibiting agents such as phenolic compounds, e.g. 2,6-di-*t*-butyl-4-methylphenol, *n*-octadecyl 3-(3,5-di-*t*-butyl-4-hydroxyphenyl)propionate or *tetrakis*[3-(3,5-di-*t*-butyl-4-hydroxyphenyl)propionyloxymethyl]methane, have hitherto been employed. However, each of these agents has a disadvantage that when incorporated in an organic substance at a high temperature for a prolonged period, it does not have a prolonged effect.

We have discovered that acetylene carbamide derivatives having a specific structure represented by the above general formula (I) have an excellent effect as antioxidants.

GB—A—2010875 discloses coating compositions containing, as cross-linking agents, compounds having the acetylene carbamide skeleton as in formula (I) above, but these prior compounds do not have any aryl groups in the side chains, and the carbamide skeletons are linked by —CH— groups; there is no suggestion that these prior agents are useful as stabilizers in resins or for any purpose except for cross-linking of polyols.

The acetylene carbamide derivatives of the above general formula (I) are novel compounds which have been synthesized by the present inventors for the first time; they can be produced from, as starting material, an acetylene carbamide of the general formula (II):

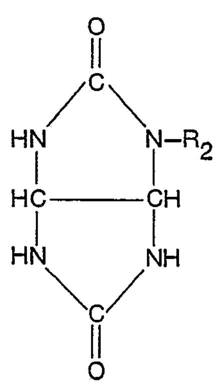

(II)

wherein $R_2$ represents a hydrogen atom or a methyl group, by the following processes:

(A) A process which comprises reacting an acetylene carbamide of the general formula (II) and a *p*-hydroxybenzyl alcohol derivative of the general formula (III):

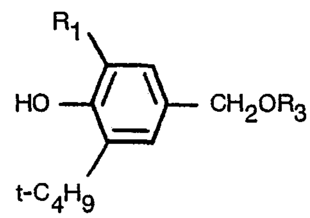

(III)

wherein $R_1$ is as defined above, and $R_3$ represents a hydrogen atom or an alkyl group having 1 to 4 carbon atoms, in a solvent in the presence of a catalyst.

(B) A process which comprises reacting an acetylene carbamide derivative of the general formula (II) and a dialkyldithiocarbamate derivative of the general formula (IV):

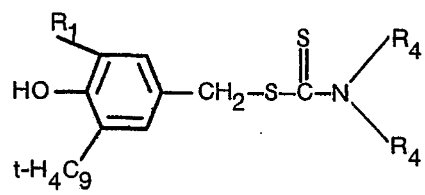

(IV)

wherein $R_1$ is as defined above, and $R_4$ represents an alkyl group having 1 to 4 carbom atoms, in a solvent in the presence of a catalyst.

(C) A process which comprises simultaneously reacting an acetylene carbamide of the general formula (II), formaldehyde and a phenol of the general formula (V):

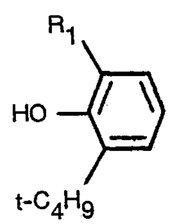

(V)

wherein $R_1$ is as defined above, in a solvent in the presence of a catalyst.

In the starting compounds used in the above-described respective processes, examples of the *p*-hydroxybenzyl alcohol derivative of the general formula (III) include 3-*t*-butyl-4-hydroxybenzyl alcohol, 3-methyl-5-*t*-butyl-4-hydroxybenzyl alcohol, 3,5-di-*t*-butyl-4-hydroxybenzyl alcohol, 3-propyl-5-*t*-butyl-4-hydroxybenzyl alcohol, 3-*t*-butyl-4-hydroxybenzyl methyl ether, 3-methyl-5-*t*-butyl-4-hydroxybenzyl methyl ether, 3,5-di-*t*-butyl-4-hydroxybenzyl methyl ether and 3,5-di-*t*-butyl-4-hydroxybenzyl butyl ether; examples of the dialkyldithiocarbamate derivative of the general formula (IV) include 3-*t*-butyl-4-hydroxybenzyl-N,N-dimethyldithiocarbamate, 3-methyl-5-*t*-butyl-4-hydroxybenzyl-N,N-diethyldithiocarbamate and 3,5-di-*t*-butyl-4-hydroxybenzyl-N,N-dimethyldithiocarbamate; and examples of the phenol of the general formula (V) include 2-*t*-butylphenol, 2-methyl-6-*t*-butylphenol and 2,6-di-*t*-butylphenol.

In each reaction of the above processes (A) to (C), the molar ratio of the respective reactants varies slightly depending on whether the acetylene carbamide of the general formula (II) used as the starting material is acetylene carbamide (i.e., the substituent $R_2$ is a hydrogen atom) or N-methylacetylene carbamide (i.e., the substituent $R_2$ is a methyl group). This is because of the difference in number of

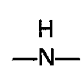

moiety which contributes to the reaction of the acetylene carbamide.

In the process (A), the molar ratio of the *p*-hydroxybenzyl alcohol derivative of acetylene carbamide is generally 3.5:1 to 6:1, preferably 4:1 to 5:1, more preferably 4.2:1 to 4.6:1, whereas the molar ratio of the *p*-

hydroxybenzyl alcohol derivative to N-methylacetylene carbamide is generally 2.5:1 to 5:1, preferably 3:1 to 4:1.

In the process (B), the molar ratio of the dialkyldithiocarbamate derivative to acetylene carbamide is generally 3.5:1 to 5:1, preferably 4:1 to 4.6:1, whereas the molar ratio of the dialkyldithiocarbamate derivative to N-methylacetylene carbamide is generally 2.5:1 to 4.5:1, preferably 3:1 to 4:1.

In the process (C), the molar ratio of acetylene carbamide to the phenol to formaldehyde is generally 1:3.5 to 8:3.5 to 8, preferably 1:4 to 6:4 to 6, whereas the molar ratio of N-methylacetylene carbamide to the phenol to formaldehyde is generally 1:2.5 to 6:2.5 to 6, preferably 1:3 to 4.5:3 to 4.5.

In each reaction of the above processes (A) to (C), there can be employed as the solvent lower alcohols such as methanol, ethanol, *n*-propyl alcohol, isopropyl alcohol, *n*-butyl alcohol, isobutyl alcohol or *sec*-butyl alcohol, with methanol and ethanol being preferable. Further, together with these alcohols, it is also possible to use other organic solvents such as aliphatic hydrocarbons, e.g. *n*-hexane or *n*-heptane; alicyclic hydrocarbons, e.g. cyclohexane; aromatic hydrocarbons, e.g. benzene, toluene or xylene; halogenated hydrocarbons, e.g. chloroform or carbon tetrachloride; and aprotonic polar solvents, e.g. N,N'-dimethylformamide or dimethyl sulfoxide.

While the reaction temperature varies depending on the kind of solvent used, it is generally 20°C to 200°C, preferably 40° to 150°C. The reaction, however, is generally carried out at the reflux temperature.

Examples of the catalyst which can be used include a basic catalyst, such as an alkali metal hydroxide, e.g. lithium hydroxide, sodium hydroxide or potassium hydroxide; an alkali metal alkoxide, e.g. sodium methoxide, potassium methoxide, sodium ethoxide, potassium ethoxide or potassium *t*-butoxide. The amount of the catalyst to be used also slightly varies depending on whether the starting material is acetylene carbamide or N-methylacetylene carbamide, similarly to variation of the molar ratio of the respective reactants.

Specifically, when acetylene carbamide is employed, the amount of the catalyst is generally 0.1 to 6 moles, preferably 4 to 5 moles, in the process (A); generally 3.5 to 12 moles, preferably 8 to 10 moles, in the process (B); and generally 0.1 to 9 moles, preferably 4 to 6 moles, in the process (C), per mole of acetylene carbamide, respectively. On the other hand, when N-methylacetylene carbamide is employed, the amount of the catalyst is generally 0.04 to 5 moles, preferably 3 to 4 moles, in the process (A); generally 2.5 to 9 moles, preferably 5 to 7.5 moles, in the process (B); and generally 0.1 to 7 moles, preferably 3 to 4.5 moles, in the process (C), per mole of N-methylacetylene carbamide, respectively.

Separation of the desired product from each reaction mixture obtained by the processes (A) to (C) can be achieved by a conventional method, e.g. (1) a method in which if desired, an acid is added to the reaction mixture to neutralize the catalyst, the solvent is distilled off, a solvent which is insoluble or sparingly soluble in water but which can dissolve therein the desired product, such as toluene or ethyl acetate is added to the concentrate to thereby extract the desired product, and the extract is washed with water and then concentrated, or (2) a method in which the basic catalyst present in the reaction mixture is neutralized, and precipitated crystals are separated by filtration with or without the sovlent having been removed. The thus separated desired product can be purified by a conventional method e.g. further recrystallization or solvent-washing.

Specific acetylene carbamide derivative of the general formula (I) of the present invention are listed below:

(1) N,N',N'',N'''-*Tetrakis*(3-*t*-butyl-4-hydroxybenzyl)acetylene carbamide
(2) N,N',N'',N'''-*Tetrakis*(3-methyl-5-*t*-butyl-4-hydroxybenzyl)acetylene carbamide
(3) N,N',N'',N'''-*Tetrakis*(3-ethyl-5-*t*-butyl-4-hydroxybenzyl)acetylene carbamide
(4) N,N',N'',N'''-*Tetrakis*(3-*n*-propyl-5-*t*-butyl-4-hydroxybenzyl)acetylene carbamide
(5) N,N',N'',N'''-*Tetrakis*(3,5-di-*t*-butyl-4-hydroxybenzyl)acetylene carbamide
(6) N,N',N''-*Tris*(3-*t*-butyl-4-hydroxybenzyl)-N'''-methylacetylene carbamide
(7) N,N',N''-*Tris*(3-methyl-5-*t*-butyl-4-hydroxybenzyl)-N'''-methylacetylene carbamide
(8) N,N',N''-*Tris*(3-ethyl-5-*t*-butyl-4-hydroxybenzyl)-N'''-methylacetylene carbamide
(9) N,N',N''-*Tris*(3-isopropyl-5-*t*-butyl-4-hydroxybenzyl)-N'''-methylacetylene carbamide
(10) N,N',N''-*Tris*(3,5-di-*t*-butyl-4-hydroxybenzyl)-N'''-methylacetylene carbamide.

These acetylene carbamide derivatives are effective as antioxidants for synthetic resins such as polyolefins, acrylonitrile-butadiene-styrene copolymers (ABS resins), polystyrene, high impact polystyrene, polyamides, polyacetals and ethylene-propylene copolymers; natural rubbers; synthetic rubbers such as butadiene rubber, isoprene rubber, styrene-butadiene copolymer rubbers, acrylonitrile-butadiene rubbers and terpolymers of ethylene, propylene and a diene; petroleum products such as lubricants or fuel oils; and various other organic substances. In particular, the acetylene carbamide derivatives of the present invention are effective against the oxidative deterioration of polyolefin resins, styrenic resins and synthetic rubbers produced by the solution polymerization process, and *inter alia*, polypropylene resins, ABS resins and butadiene rubber are most effectively protected.

When the acetylene carbamide derivatives of this invention are used as antioxidants, the amount to be used varies depending on the kind of organic substance to be stabilized, but it is generally 0.001 to 10% by weight based on the weight of the organic substance, and in many applications it is 0.01 to 5% by weight based on the amount of the organic substance.

To incorporate the acetylene carbamide derivative of this invention into an organic substance,

conventional known equipment and methods can be employed.

While the acetylene carbamide derivatives of this invention are effective as antioxidants for organic substances even when used alone, an excellent synergistic effect can be obtained when they are used together with a sulfur-based antioxidant such as a dialkyl-3,3'-thiodipropionate of the general formula (VI):

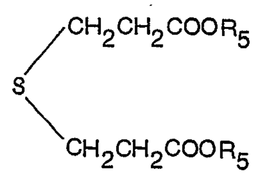

(VI)

wherein $R_5$ is an alkyl group having 12 to 20 carbon atoms, e.g. dilauryl-3,3'-thiodipropionate, dimyristyl-3,3'-thiodipropionate or distearyl-3,3'-thiodipropionate; a pentaerythritol-tetrakis(β-alkylthiopropionate) of the general formula (VIII):

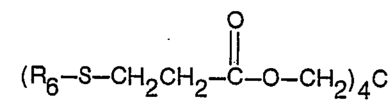

(VIII)

wherein $R_6$ is an alkyl group having 4 to 20, preferably 12 to 20, carbon atoms, e.g. pentaerythritol-tetrakis(β-laurylthiopropionate) or pentaerythritol-tetrakis(β-stearylthiopropionate); or distearyl disulfide.

When the sulfur-based antioxidant is thus also used, the amount thereof is generally 0.5 to 15 times the weight of the acetylene carbamide derivative of this invention, and the total amount of the acetylene carbamide derivative and the sulfur-based antioxidant is preferably 0.01 to 10 parts by weight per 100 parts by weight of the organic substance.

Furthermore, the light resistance can be improved by incorporating thereinto an ultraviolet absorber, an organonickel light stabilizer or a hindered amine light stabilizer, such as 2-(2-hydroxy-4-octoxyphenyl)benzotriazole, 2-(2-hydroxy-5-methylphenyl)benzotriazole, 2-(2-hydroxy-3,5-di-*t*-butylphenyl)-5-chlorobenzotriazole, 2-(2-hydroxy-3,5-di-*t*-butylphenyl)benzotriazole, 2-(2-hydroxy-3-*t*-butyl-5-methylphenyl)-5-chlorobenzotriazole, 2-(2-hydroxy-3-*t*-butyl-5-methylphenyl)benzotriazole, 2-(2-hydroxy-3,5-di-*t*-amylphenyl)-5-chlorobenzotriazole, 2-(2-hydroxy-3,5-di-*t*-amylphenyl)benzotriazole, 2-(2-hydroxy-5-*t*-octylphenyl)benzotriazole, 2,4-dihydroxybenzophenone, 2-hydroxy-4-methoxybenzophenone, 2-hydroxy-4-octoxybenzophenone, 2-hydroxy-4-dodecyloxybenzophenone, 2-hydroxy-4-benzyloxybenzophenone, 2,4,2',4'-tetrahydroxybenzophenone, 2,2'-dihydroxy-4,4'-dimethoxybenzophenone, [2,2'-thio*bis*(4-*t*-octylphenolato)]-*n*-butylamine nickel (II), [2,2'-thio*bis*(4-*t*-octylphenolato)]triethanolamine nickel (II), [2,2'-thiobis(4-*t*-octylphenolato)]cyclohexyldiethanolamine nickel (II), *bis*(3,5-di-*t*-butyl-4-hydroxybenzylphosphoric acid) monoethyl ester nickel salt, *bis*(2,2,6,6-tetramethyl-4-piperidyl)-sebacate, 4-benzoyloxy-2,2,6,6-tetramethylpiperidine, *bis*(2,2,6,6-tetramethyl-4-piperidinyl)-*para*-phenylenediamine, *bis*(1,2,2,6,6-pentamethylpiperidin-4-yl) 2-(3,5-di-*t*-butyl-4-hydroxybenzyl)-2-*n*-butyl malonate, 1-(2-[3,5-di-*t*-butyl-4-hydroxyphenyl)propionyloxy]-ethyl)-4-[3,5-di-*t*-butyl-4-hydroxyphenyl)propionyloxy]-2,2,6,6-tetramethylpiperidine, dimethyl succinate-1-(2-hydroxyethyl)-4-hydroxy-2,2,6,6-tetramethylpiperidine polycondensate or 1,4-bis(2,2,6,6-tetramethylpiperidin-4-carbonyloxymethyl)cyclohexane.

Still further, the color hue can be enhanced by incorporating a phosphorus-based antioxidant such as *tris*(mono- and/or di-nonylphenyl) phosphite, *tris*(2,4-di-*t*-butylphenyl) phosphite, *tris*(2-*t*-butyl-4-methylphenyl) phosphite, *tetrakis*(2,4-di-*t*-butylphenyl)-4,4'-biphenylene phosphite, 3,9-*bis*(octadecyloxy)-2,4,8,10-tetraoxa-3,9-diphosphor*spiro*[5,5]-undecane, 3,9-*bis*(dodecyloxy)-2,4,8,10-tetraoxa-3,9-diphosphor*spiro*[5,5]undecane, 3,9-*bis*(octoxy)-2,4,8,10-tetraoxa-3,9-diphosphorspiro[5,5]undecane or *tetrakis*(2,4-di-*t*-butylphenyl)[1,1'-biphenyl]-4,4'-diylbisphosphonite.

In addition to the above, other antioxidants and light stabilizers, as well as plasticizers, nucleating agents, lubricating agents, antistatic agents, flame retardants, metal inactivating agents, metal soaps, pigments and dyes, fillers, anticorrosives, rust preventives, pour point depressants, defoaming agents, dispersants, or extreme pressure additives can be incorporated depending upon the respective purposes, if desired.

When such various additives are employed in combination, they can be mixed with the acetylene carbamide derivative of the present invention in advance, and there is no specific limitation on their combined use.

The present invention is more particularly described by the following examples.

Example 1

Into a 300 ml four-necked flask equipped with a thermometer, a stirrer and a condenser were charged 40.36 g (0.138 mole) of 3,5-di-*t*-butyl-4-hydroxybenzyl butyl ether, 4.26 g (0.03 mole) of acetylene

carbamide and 50 ml of butanol. After purging the flask with nitrogen, 8.60 g (138 mol) of 85 wt% potassium hydroxide was added thereto, the temperature was raised, and the reaction was carried out under reflux conditions for 3 hours. After completion of the reaction, the reaction mixture was treated with 75 ml (0.15 mole) of 2N hydrochloric acid and then extracted with 100 ml of toluene. The extracted toluene layer was washed with water and then concentrated under reduced pressure, and 50 ml of *n*-hexane was added to the concentrate to thereby subject it to recrystallization, to obtain 28.94 g (yield: 95%) of N,N',N'',N'''-*tetrakis*(3,5-di-*t*-butyl-4-hydroxybenzyl)acetylene carbamide as white crystals having a melting point of 244 to 246°C.

Elemental analysis for $C_{64}H_{94}N_4O_6$ (calculated values in parentheses):

C: 75.30% (75.70%)
H: 9.45% (9.33%)
N: 5.46% (5.52%)

FD—MS: Molecular ion peak: 1014

$^1H$—NMR ($CDCl_3$, TMS):

δ1.37 72H, s
δ4.10 4H, d, J=15 Hz
δ4.57 4H, d, J=15 Hz
δ4.96 2H, s
δ5.14 8H, s
δ6.97 8H, s

## Example 2

Into a 200 ml four-necked flask equipped with a thermometer, a stirrer and a condenser were charged 9.57 g (0.046 mole) of 3-*t*-butyl-5-methyl-4-hydroxybenzyl methyl ether, 1.42 g (0.010 mole) of acetylene carbamide and 20 ml of methanol. After purging the flask with nitrogen, 8.87 g (0.046 mole) of a 28 wt% solution of sodium methoxide in methanol was added thereto, the temperature was raised, and the reaction was carried out under reflux condition for 40 hours. After completion of the reaction, the reaction mixture was treated with 30 ml (0.06 mole) of 2N hydrochloric acid, and then extracted with 100 ml of ethyl acetate. The extracted ethyl acetate layer was treated in the same manner as in Example 1 to obtain 7.87 g (yield: 93%) of N,N',N'',N'''-*tetrakis*(3-*t*-butyl-5-methyl-4-hydroxybenzyl)acetylene carbamide as white crystals having a melting point of 225°C to 227°C.

Elemental analysis for $C_{52}H_{70}N_4O_6$ (calculated values in parentheses):

C: 73.91% (73.77%)
H: 8.35% (8.28%)
N: 6.58% (6.62%)

FD—MS: Molecular ion peak: 846

$^1H$—NMR ($CDCl_3$, TMS):

δ1.33 36H, s
δ2.12 12H, s
δ3.97 4H, d, J=15 Hz
δ4.80 4H, s
δ4.81 4H, d, J=15 Hz
δ4.87 2H, s
δ6.64 4H, br. s
δ6.93 4H, br. s

## Example 3

Into the same reactor as used in Example 1 were charged 8.51 g (0.034 mole) of 3,5-di-*t*-butyl-4-hydroxybenzyl methyl ether, 1.56 g (0.01 mole) of N-methylacetylene carbamide and 50 ml of methanol. After purging the reactor with nitrogen, 6.56 g (0.035 mole) of a 28 wt% solution of sodium methoxide in methanol was added thereto, the temperature was raised, and the reaction was carried out under reflux condition for 5 hours. After completion of the reaction, the reaction mixture was treated with 40 ml (0.04 mole) of 1N hydrochloric acid, and then post-treated in the same manner as in Example 1 to obtain 7.62 g (yield: 94%) of glasslike, N,N',N''-*tris*(3,5-di-*t*-butyl-4-hydroxybenzyl)methylacetylene carbamide.

## Example 4

N,N',N'',N'''-*tetrakis*(3-*t*-butyl-4-hydroxybenzyl)acetylene carbamide was obtained by repeating the procedure of Example 2, from 3-*t*-butyl-4-hydroxybenzyl alcohol and acetylene carbamide with 28 wt% solution of sodium methoxide in methanol as a catalyst.

## Example 5

In to same reactor as used in Example 1 was charged 15.62 g (0.046 mole) of 3,5-di-*t*-butyl-4-hydroxybenzyl-dimethyldithiocarbamate, 1.42 g (0.01 mole) of acetylene carbamide and 50 ml of methanol. After purging the reactor with nitrogen, 6.59 g (0.10 mole) of 85 wt% potassium hydroxide was added thereto as a catalyst, and the reaction was carried out under reflux condition for 3 hours. After completion of the

reaction, the reaction mixture was post-treated in the same manner as in Example 1 to separate and purify the product. Thus, 9.85 g (yield: 97%) of N,N',N'',N'''-*tetrakis*(3,5-di-*t*-butyl-4-hydroxybenzyl)acetylene carbamide was obtained as white crystals.

Example 6

In the same manner as in Example 3, 14.97 g (0.046 mole) of 3-*t*-butyl-5-methyl-4-hydroxybenzyl-diethyldithiocarbamate and 1.42 g (0.01 mole) of acetylene carbamide were reacted under reflux condition in a methanol solvent in the presence of 19.28 g (0.01 mole) of a 28 wt% solution of sodium methoxide in methanol as a catalyst for 10 hours, and then post-treated in the same manner as in Example 2 to obtain 7.79 g (yield: 92%) of N,N',N'',N'''-*tetrakis*(3-*t*-butyl-5-methyl-4-hydroxybenzyl)acetylene carbamide as white crystals.

Example 7

Into the same reactor as used in Example 1 were charged 11.88 g (0.035 mole) of 3,5-di-*t*-butyl-4-hydroxybenzyl-dimethyldithiocarbamate, 1.56 g (0.01 mole) of N-methylacetylene carbamide and 50 ml of methanol. After purging the reactor with nitrogen, 4.61 g (0.07 mole) of 85 wt% potassium hydroxide was added thereto, the temperature was elevated, and the reaction was carried out under reflux condition for 3 hours. After completion of the reaction, the reaction mixture was neutralized with 70 ml (0.07 mole) of 1N hydrochloric acid, and post-treated in the same manner as in Example 1 to obtain 7.70 g (yield: 95%) of glass-like N,N',N''-*tris*(3,5-di-*t*-butyl-4-hydroxybenzyl)-N'''-methylacetylene carbamide.

Example 8

Into the same reactor as used in Example 2 were charged 11.55 g (0.056 mole) of 2,6-di-*t*-butylphenol, 1.42 g (0.01 mole) of acetylene carbamide, 3.65 g (0.056 mole) of a 46 wt% solution of formaldehyde in methanol, 25 ml of methanol and 25 ml of *n*-hexane. After purging the reactor with nitrogen, 3.69 g (0.056 mole) of 85 wt% potassium hydroxide was added thereto, the temperature was elevated, and the reaction was carried out under reflux condition for 10 hours. After completion of the reaction, the reaction mixture was treated with 30 ml (0.06 mole) of 2N hydrochloric acid, and post-treated in the same manner as in Example 1 to obtain 9.75 g (yield: 96%) of N,N',N'',N'''-*tetrakis*(3,5-di-*t*-butyl-4-hydroxybenzyl)acetylene carbamide as white crystals.

Example 9

Into the same reactor as used in Example 1 were charged 8.67 g (0.042 mole) of 2,6-di-*t*-butylphenol, 1.56 g (0.01 mole) of N-methylacetylene carbamide, 2.74 g (0.042 mole) of a 46 wt% solution of formaldehyde in methanol, 25 ml of methanol and 25 ml of *n*-hexane. After purging the reactor with nitrogen, 2.77 g (0.042 mole) of 85 wt% potassium hydroxide was added thereto, the temperature was raised, and the reaction was carried out under reflux condition for 10 hours. After completion of the reaction, the reaction mixture was neutralized with 4.12 g (0.042 mole) of phosphoric acid, and post-treated in the same manner as in Example 1 to obtain 7.70 g (yield: 95%) of glass-like N,N',N''-*tris*(3,5-di-*t*-butyl-4-hydroxybenzyl)-N'''-methylacetylene carbamide.

Example 10

The same procedure as in Example 8 was repeated except that the 2,6-di-*t*-butylphenyl was replaced by 2-*t*-butyl-6-methylphenol. The product was isolated from the reaction mixture to obtain N,N',N'',N'''-*tetrakis*(3-*t*-butyl-5-methyl-4-hydroxybenzyl)acetylene carbamide.

Example 11

Into a 200 ml four-necked flask equipped with a water separator, a thermometer and a stirrer were charged 6.24 g (0.027 mole) of 3,5-di-*t*-butyl-4-hydroxybenzyl alcohol, 0.84 g (0.006 mole) of acetylene carbamide and 50 g of toluene. After purging the reactor with nitrogen, 1.0 g (0.005 mole) of a 28 wt% solution and sodium methoxide in methanol was added thereto, and the reaction was carried out under reflux condition for 5 hours. After completion of the reaction, the reaction mixture was neutralized with 2.5 ml (0.005 mole) of 2N hydrochloric acid, and post-treated in the same manner as in Example 1 to obtain 3.5 g (yield: 58%) of N,N',N'',N'''-*tetrakis*(3,5-di-*t*-butyl-4-hydroxybenzyl)acetylene carbamide as white crystals.

Example 12

Into the same flask as used in Example 11 were charged 5.8 g (0.03 mole) of 3-*t*-butyl-5-methyl-4-hydroxybenzyl alcohol, 0.84 g (0.006 mole) of acetylene carbamide and 30 g of *p*-xylene. After purging the reactor with nitrogen, 2.0 g (0.008 mole) of a 28 wt% solution of potassium methoxide in methanol was added thereto, and the reaction was carried out at 140°C for 8 hours. After completion of the reaction, the reaction mixture was neutralized with 4 ml (0.008 mole) of 2N hydrochloric acid, and post-treated in the same manner as in Example 2 to obtain 2.8 g (yield: 55%) of N,N',N'',N'''-*tetrakis*(3-*t*-butyl-5-methyl-4-hydroxybenzyl)acetylene carbamide as white crystals.

Example 13

N,N',N'',N'''-*tetrakis*(3-*t*-butyl-4-hydroxybenzyl)acetylene carbamide was obtained by repeating the same procedure as in Example 11 from *3-t*-butyl-4-hydroxybenzyl alcohol and acetylene carbamide with toluene as a solvent and a 28 wt% solution of sodium methoxide in methanol as a catalyst being used, respectively.

Example 14

Into a 200 ml four-necked flask equipped with a thermometer, a stirrer and a condenser were charged 11.7 g (0.05 mole) of 3,5-di-*t*-butyl-4-hydroxybenzyl alcohol, 2.34 g (0.015 mole) of N-methylacetylene carbamide, 25 ml of methanol and 25 ml of *n*-hexane. After purging the reactor with nitrogen, 1.65 g (0.025 mole) of 85 wt% potassium hydroxide was added thereto, the temperature was elevated, and the reaction was carried out under reflux condition for 6 hours. After completion of the reaction, the reaction mixture was neutralized with 25 ml (0.025 mole) of 1N hydrochloric acid to separate a pale yellow viscous liquid. The thus separated viscous liquid was extracted with 100 ml of toluene, and washed with water. The toluene layer was distilled off under reduced pressure, and the distillation residue was washed with 50 ml of hot *n*-hexane and then cooled to obtained 11 g (yield: 91%) of pale yellow glass-like N,N',N''-*tris*(3,5-di-*t*-butyl-4-hydroxybenzyl)-N'''-methylacetylene carbamide.

Elemental analysis for $C_{50}H_{74}N_4O_5$ (calculated values in parentheses):

C: 73.71% (74.04%)
H: 9.23% (9.20%)
N: 7.02% (6.91%)

FD—MS: Molecular ion peak: 810

$^1$H—NMR ($CDCl_3$, TMS):

δ1.42 54H, s
δ2.8 3H, s
δ4.0—5.0 8H, m
δ5.2 3H, s
δ6.9—7.1 6H, br

Example 15

The same procedure as in Example 14 was repeated except that the 3,5-di-*t*-butyl-4-hydroxybenzyl alcohol was replaced by 3-*t*-butyl-5-methyl-4-hydroxybenzyl alcohol. The product was isolated from the reaction mixture to obtain N,N',N''-*tris*(3-*t*-butyl-5-methyl-4-hydroxybenzyl)-N'''-methylacetylene carbamide.

The following examples show the use of compounds of the invention, and comparison compounds, as antioxidants.

Example 16

Each of the following formulations were mixed in a mixer for 5 minutes, and melted and kneaded by means of a mixing roll at 180°C to obtain a mixture which was then molded into a sheet of 1 mm in thickness using a hot press at 210°C. Thus, a test piece of size 40 × 10 × 1 mm was prepared. The time to when the test piece had become brittle to an extend of 30% of the surface area thereof was measured in a Geer's oven at 160°C, and this was designated as a thermal brittleness induction period. Thus, stability against heat and oxidation was evaluated. The results obtained are given in Table 1.

*Formulation*

Non-stabilized Polypropylene Resin: 100 parts by weight
Calcium Stearate: 1 part by weight
Test Compound: As indicated in Table 1.

The symbols under the test compounds in Table 1 stand for the following compounds, respectively.

I-1: N,N',N'',N'''-*Tetrakis*(3,5-di-*t*-butyl-4-hydroxybenzyl)acetylene Carbamide
I-2: N,N',N'',N'''-*Tetrakis*(3-*t*-butyl-5-methyl-4-hydroxybenzyl)acetylene Carbamide
I-3: N,N',N''-*Tris*(3,5-di-*t*-butyl-4-hydroxybenzyl)-N'''-methylacetylene Carbamide
AO-1: *n*-Octadecyl 3-(3,5-di-*t*-butyl-4-hydroxybenzyl)propionate
AO-2: 1,1,3-*Tris*(3-*t*-butyl-6-methyl-4-hydroxyphenyl)butane
AO-3: *Tetrakis*[3-(3,5-di-*t*-butyl-4-hydroxyphenyl)pripionyloxymethyl]methane
AO-4: Dimyristyl 3,3'-Thiodipropionate
AO-5: Pentaerythritol-*tetrakis*(β-laurylthiopropionate)

TABLE 1

| Test Compound | Present Invention Run No. | | | | | | | | | | |
|---|---|---|---|---|---|---|---|---|---|---|---|
| | 1 | 2 | 3 | 4 | 5 | 6 | 7 | 8 | 9 | 10 | 11 |
| Phenol Type | | | | | | | | | | | |
| I-1 | 0.05 | | | 0.05 | 0.05 | | | | | | |
| I-2 | | 0.05 | | | | 0.05 | 0.05 | 0.05 | 0.05 | | |
| I-3 | | | 0.05 | | | | | | | 0.05 | 0.05 |
| AO-1 | | | | | | | | | | | |
| AO-2 | | | | | | | | | | | |
| AO-3 | | | | | | | | | | | |
| Sulfur Type | | | | | | | | | | | |
| AO-4 | | | | 0.2 | | 0.2 | 0.4 | | | 0.2 | |
| AO-5 | | | | | 0.2 | | | 0.2 | 0.4 | | 0.4 |
| Brittleness Induction Period (hrs) | 80 | 75 | 70 | 760 | 730 | 730 | 910 | 1470 | 2020 | 690 | 680 |

TABLE 1 (continued)

| Test Compound | Comparison Run No. | | | | | | | | | | | |
|---|---|---|---|---|---|---|---|---|---|---|---|---|
| | 12 | 13 | 14 | 15 | 16 | 17 | 18 | 19 | 20 | 21 | 22 | 23 |
| Phenol Type I-1 | | | | | | | | | | | | |
| I-2 | | | | | | | | | | | | |
| I-3 | | | | | | | | | | | | NO |
| AO-1 | 0.05 | | | 0.05 | 0.05 | | | | | | | ADDITION |
| AO-2 | | 0.05 | | | | 0.05 | 0.05 | | | | | |
| AO-3 | | | 0.05 | | | | | 0.05 | 0.05 | 0.05 | 0.05 | |
| Sulfur Type | | | | | | | | | | | | |
| AO-4 | | | | 0.2 | | 0.2 | | 0.2 | 0.4 | | | |
| AO-5 | | | | | 0.2 | | 0.2 | | | 0.2 | 0.4 | |
| Brittleness Induction Period (hrs) | 30 | 25 | 50 | 320 | 310 | 380 | 390 | 490 | 610 | 400 | 550 | 5 |

Example 17

100 parts by weight of a non-stabilized ABS resin powder was added to a grinder and dispersed in an appropriate amount of methanol. 0.5 Part by weight of each test compound was added thereto, and the methanol was evaporated while intimately mixing the mixture. The thus-obtained ABS resin powder was used as a test sample. Stability against heat and oxidation was evaluated in terms of degree of discoloration of the ABS resin powder after ageing in a Geer's oven at 180°C. The results obtained are given in Table 2.

The symbols for the test compounds used in Table 2 have the same meanings as those in Example 16.

TABLE 2

| Run No. | Test Compound | Amount Added (pts. by weight) | Degree of Discoloration After 30 Min. | Degree of Discoloration After 60 Min. |
|---|---|---|---|---|
| Present Invention | | | | |
| 1 | I-1 | 0.5 | Pale yellow | Yellowish brown |
| 2 | I-2 | 0.5 | " | " |
| 3 | I-3 | 0.5 | " | " |
| 4 | I-1/AO-4 | 0.2/0.3 | " | " |
| 5 | I-1/AO-5 | 0.2/0.3 | " | " |
| 6 | I-2/AO-4 | 0.2/0.3 | " | " |
| 7 | I-2/AO-4 | 0.1/0.4 | " | " |
| 8 | I-2/AO-4 | 0.2/0.3 | " | " |
| 9 | I-2/AO-5 | 0.1/0.4 | " | " |
| 10 | I-3/AO-4 | 0.2/0.3 | " | " |
| 11 | I-3/AO-5 | 0.2/0.2 | " | " |
| Comparison | | | | |
| 12 | AO-1 | 0.5 | Yellowish brown | Deep brown |
| 13 | AO-2 | 0.5 | " | " |
| 14 | AO-3 | 0.5 | " | Brown |
| 15 | AO-1/AO-4 | 0.2/0.3 | " | Deep brown |
| 16 | AO-1/AO-5 | 0.2/0.3 | " | " |
| 17 | AO-2/AO-4 | 0.2/0.3 | " | " |
| 18 | AO-2/AO-5 | 0.2/0.3 | " | " |
| 19 | AO-3/AO-4 | 0.2/0.3 | " | Brown |
| 20 | AO-3/AO-4 | 0.1/0.4 | " | " |

TABLE 2 (Cont'd)

| Run No. | Test Compound | Amount Added | Degree of Discoloration | |
|---|---|---|---|---|
| | | | After 30 Min. | After 60 Min. |
| Comparison | | (pts. by weight) | | |
| 21 | AO-3/AO-5 | 0.2/0.3 | Yellowish brown | Brown |
| 22 | AO-3/AO-5 | 0.1/0.4 | " | " |
| 23 | No Addition | | Dark brown | Dark brown |

Example 18

Polybutadiene rubber produced by the solution polymerization process and containing no antioxidant (a rubber prepared by extracting a commercially available product, JSR BR-01 with acetone to remove the antioxidant was used) was mixed with each test compound on a roll kneader. The thus-obtained rubber was used as a test rubber, and tests on the stability against heat and oxidation and the resistance to heat discoloration were carried out. The stability against heat and oxidation was evaluated in terms of time to when the gel content had reached 10% by weight (designated as Gel I.P.) by subjecting the test rubber to ageing by heat in a Geer's oven at 100°C and measuring the gel content (i.e., toluene-insoluble matter) at 15 hours intervals.

The resistance to heat discoloration was evaluated in terms of color hue of rubber 15 hours, 60 hours and 120 hours after ageing by heat in a Geer's oven at 100°C. The results obtained are given in Table 3.

In Table 3, the symbols AO-6 and AO-7 for the test compounds stand for the compounds given below, and the rest of the symbols have the same meanings as those in Example 16.

AO-6: *Tris*(nonylphenyl) Phosphite

AO-7: Distearyl Pentaerythritol Diphosphite

TABLE 3

| Test Compound | Present Invention Run No. 1 | 2 | 3 | 4 | 5 | 6 | 7 | 8 | 9 | 10 | 11 |
|---|---|---|---|---|---|---|---|---|---|---|---|
| Phenol Type | | | | | | | | | | | |
| I-1 | 1.0 | | | 0.25 | 0.25 | | | | | | |
| I-2 | | 1.0 | | | | 0.25 | 0.10 | 0.25 | 0.10 | | |
| I-3 | | | 1.0 | | | | | | | 0.25 | 0.25 |
| AO-1 | | | | | | | | | | | |
| AO-2 | | | | | | | | | | | |
| AO-3 | | | | | | | | | | | |
| Sulfur Type | | | | | | | | | | | |
| AO-4 | | | | 0.75 | | 0.75 | 0.90 | | | 0.75 | |
| AO-5 | | | | | 0.75 | | | 0.75 | 0.90 | | 0.75 |
| Phosphorus Type | | | | | | | | | | | |
| AO-6 | | | | | | | | | | | |
| AO-7 | | | | | | | | | | | |
| Gel I.P. (hrs.) | 155 | 135 | 130 | 160 | 160 | 155 | 150 | 190 | 175 | 150 | 145 |
| Resistance to Heat Discoloration | | | | | | | | | | | |
| 0 Hr. | White | White | White | White | White | White | White | White | White | White | White |
| 15 Hrs. | " | " | " | " | " | " | " | " | " | " | " |
| 60 Hrs. | " | " | " | " | " | " | " | " | " | " | " |
| 120 Hrs. | " | " | " | " | " | " | " | " | " | " | " |

(cont'd)

TABLE 3 (cont'd)

| Test Compound | Comparison Run No. | | | | | | | | | | |
|---|---|---|---|---|---|---|---|---|---|---|---|
| | 12 | 13 | 14 | 15 | 16 | 17 | 18 | 19 | 20 | 21 | 22 |
| Phenol Type | | | | | | | | | | | |
| I-1 | | | | | | | | | | | |
| I-2 | | | | | | | | | | | |
| I-3 | | | | | | | | | | | |
| AO-1 | 1.0 | | | | | | | 0.25 | 0.25 | 0.25 | 0.25 |
| AO-2 | | 1.0 | | | | | | | | | |
| AO-3 | | | 1.0 | | | | | | | | |
| Sulfur Type | | | | | | | | | | | |
| AO-4 | | | | 1.0 | | | | 0.75 | | | |
| AO-5 | | | | | 1.0 | | | | 0.75 | | |
| Phosphorus Type | | | | | | | | | | | |
| AO-6 | | | | | | 1.0 | | | | 0.75 | |
| AO-7 | | | | | | | 1.0 | | | | 0.75 |
| Gel I.P. (hrs.) | 80 | 75 | 85 | 20 | 20 | 30 | 30 | 60 | 60 | 85 | 70 |
| Resistance to Heat Discoloration | | | | | | | | | | | |
| 0 Hr. | White | White | White | White | White | White | White | White | White | White | White |
| 15 Hrs. | " | " | " | Pale Yellow | Pale Yellow | " | " | " | " | " | " |
| 60 Hrs. | " | " | " | Yellow | Yellow | Yellow | Yellow | Pale Yellow | Pale Yellow | " | " |
| 120 Hrs. | Pale Yellow | Pale Yellow | Pale Yellow | " | " | " | " | Yellow | Yellow | Pale Yellow | Yellow |

(cont'd)

TABLE 3 (cont'd)

| Test Compound | Comparison Run No. | | | | | | | | | | |
|---|---|---|---|---|---|---|---|---|---|---|---|
| | 23 | 24 | 25 | 26 | 27 | 28 | 29 | 30 | 31 | 32 | 33 |
| Phenol Type | | | | | | | | | | | |
| I-1 | | | | | | | | | | | |
| I-2 | | | | | | | | | | | |
| I-3 | | | | | | | | | | | |
| AO-1 | | | | | | | | | | | NO ADDITION |
| AO-2 | 0.25 | 0.25 | 0.25 | 0.25 | | | | | | | |
| AO-3 | | | | | 0.25 | 0.10 | 0.25 | 0.10 | 0.25 | 0.25 | |
| Sulfur Type | | | | | | | | | | | |
| AO-4 | 0.75 | | | | 0.75 | 0.90 | | | | | |
| AO-5 | | 0.75 | | | | | 0.75 | 0.90 | | | |
| Phosphorus Type | | | | | | | | | | | |
| AO-6 | | | 0.75 | | | | | | 0.75 | | |
| AO-7 | | | | 0.75 | | | | | | 0.75 | |
| Gel I.P. (hrs.) | 55 | 55 | 75 | 60 | 80 | 75 | 85 | 80 | 115 | 115 | 5 |
| Resistance to Heat Discoloration | | | | | | | | | | | |
| 0 Hr. | White | White | White | White | White | White | White | White | White | White | White |
| 15 Hrs. | " | " | " | " | " | " | " | " | " | " | Yellow |
| 60 Hrs. | Pale Yellow | Pale Yellow | " | Pale Yellow | Pale Yellow | Pale Yellow | Pale Yellow | Pale Yellow | " | " | " |
| 120 Hrs. | Yellow | Yellow | Yellow | Yellow | Yellow | Yellow | Yellow | Yellow | Pale Yellow | Pale Yellow | " |

**Claims**

1. An acetylene carbamide derivative of the general formula (I):

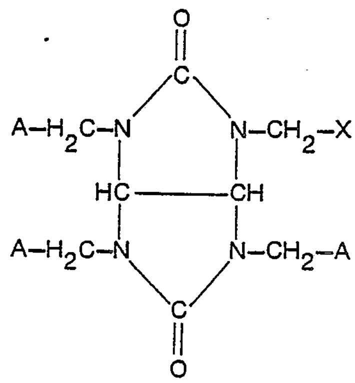

(I)

wherein each A represents the group

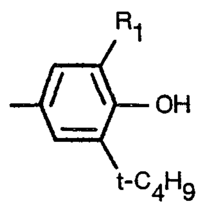

in which $R_1$ represents a hydrogen atom or an alkyl group having 1 to 4 carbon atoms and X represents A or a hydrogen atom.

2. An acetylene carbamide derivative according to Claim 1, wherein the substituent $R_1$ represents a hydrogen atom, a methyl group or a *t*-butyl group.

3. Any of the following compounds (1) to (10):

(1) N,N',N'',N'''-*Tetrakis*(3-*t*-butyl-4-hydroxybenzyl)acetylene carbamide
(2) N,N',N'',N'''-*Tetrakis*(3-methyl-5-*t*-butyl-4-hydroxybenzyl)acetylene carbamide
(3) N,N',N'',N'''-*Tetrakis*(3-ethyl-5-*t*-butyl-4-hydroxybenzyl)acetylene carbamide
(4) N,N',N'',N'''-*Tetrakis*(3-*n*-propyl-5-*t*-butyl-4-hydroxybenzyl)acetylene carbamide
(5) N,N',N'',N'''-*Tetrakis*(3,5-di-*t*-butyl-4-hydroxybenzyl)acetylene carbamide
(6) N,N',N''-*Tris*(3-*t*-butyl-4-hydroxybenzyl)-N'''-methylacetylene carbamide
(7) N,N',N''-*Tris*(3-methyl-5-*t*-butyl-4-hydroxybenzyl)-N'''-methylacetylene carbamide
(8) N,N',N''-*Tris*(3-ethyl-5-*t*-butyl-4-hydroxybenzyl)-N'''-methylacetylene carbamide
(9) N,N',N''-*Tris*(3-isopropyl-5-*t*-butyl-4-hydroxybenzyl)-N'''-methylacetylene carbamide
(10) N,N',N''-*Tris*(3,5-di-*t*-butyl-4-hydroxybenzyl)-N'''-methylacetylene carbamide

4. A process for producing an acetylene carbamide derivative as defined in Claim 1, which comprises reacting an acetylene carbamide of the general formula (II):

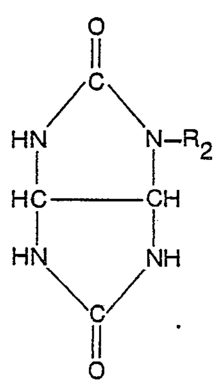

(II)

wherein $R_2$ represents a hydrogen atom or a methyl group, and a *p*-hydroxybenzyl alcohol derivative of the general formula (III):

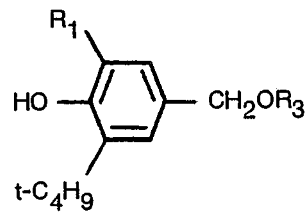

(III)

wherein $R_1$ is as in Claim 1, and $R_3$ represents a hydrogen atom or an alkyl group having 1 to 4 carbon atoms, in a solvent in the presence of a basic catalyst.

5. A process for producing an acetylene carbamide derivative as defined in Claim 1, which comprises reacting an acetylene carbamide of the general formula (II):

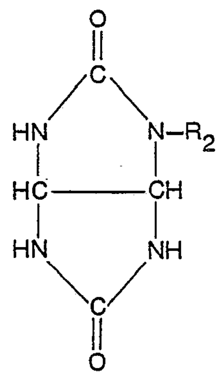

(II)

wherein $R_2$ represents a hydrogen atom or a methyl group, and a dialkyldithiocarbamate derivative of the general formula (IV):

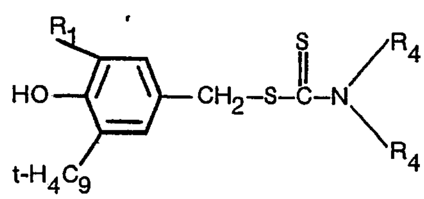

(IV)

wherein $R_1$ is as defined in Claim 1 and $R_4$ represents an alkyl group having 1 to 4 carbon atoms, in a solvent in the presence of a basic catalyst.

6. A process for producing an acetylene carbamide derivative as defined in Claim 1, which comprises simultaneously reacting an acetylene carbamide of the general formula (II) as defined in Claim 5 and a phenol of the general formula (V)

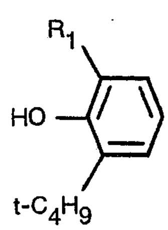

(V)

wherein $R_1$ is as defined in Claim 1, in a solvent in the presence of a basic catlayst.

7. A process according to Claim 4, 5 or 6, wherein said solvent is a lower alcohol.

8. A process according to Claim 4, 5, 6 or 7 wherein said catalyst is an alkali metal alkoxide or an alkali metal hydroxide.

9. A process according to Claim 4, 5, 6, 7 or 8, wherein the reaction is conducted at a temperature of 20 to 200°C.

10. A process according to Claim 9, wherein the reaction is conducted under reflux condition.

11. A process according to any of Claims 4 to 10, wherein the substituent $R_2$ represents a hydrogen atom and the substituent X represents the group

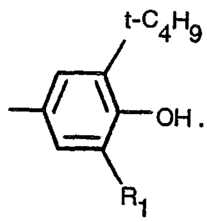

12. A process according to any of Claims 4 to 10, wherein the substituent $R_2$ is a methyl group and the substituent X is a hydrogen atom.

13. A stabilizer for organic substances which contains as an active ingredient an acetylene carbamide derivative as claimed in Claim 1, 2 or 3.

14. A stabilizer as claimed in Claim 13, which also contains a dialkyl-3,3'-thiodipropionate of the general formula (VI):

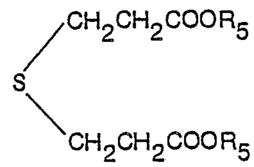

(VI)

wherein $R_5$ represents an alkyl group having 12 to 20 carbon atoms or a pentaerythritol-*tetrakis*(β-alkylthiopropionate) of the general formula (VII):

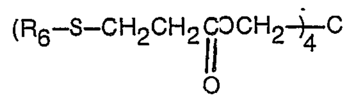

(VII)

wherein $R_6$ represents an alkyl group having 4 to 20 carbon atoms.

15. A stabilizer according to Claim 14, wherein the proportion of the acetylene carbamide derivative to the dialkyl-3,3'-thiopropionate or pentaerythritol-*tetrakis*(β-alkylthiopropionate) is 1:0.5 to 1:15 by weight.

16. An organic substance which has been stabilized by incorporating therein a stabilizer as claimed in Claim 13, 14 or 15.

17. An organic substance according to Claim 16, which is a polymer.

18. An organic substance according to Claim 17, wherein said polymer is a synthetic resin, a sythetic rubber or a natural rubber.

19. An organic substance according to Claim 18, wherein said synthetic resin a polyolefin or an acrylonitrile-butadiene-styrene copolymer.

20. An organic substance according to Claim 19, wherein said polyolefin is polypropylene.

21. An organic substance according to Claim 18, wherein said synthetic rubber is butadiene rubber.

**Patentansprüche**

1. Acetylencarbamid-Derivate der allgermeinen Formel (I)

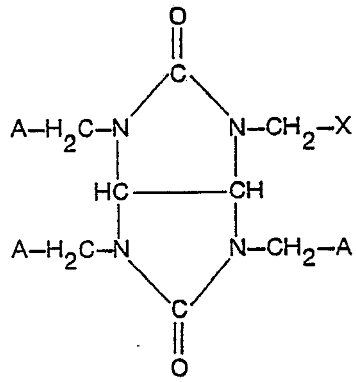

(I)

in der bedeuten:

A ein Gruppe der Formel

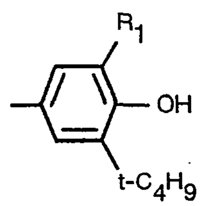

$R_1$ Wasserstoff oder $C_{1-4}$-Alkyl und

X A oder Wasserstoff.

2. Acetylencarbamid-Derivate nach Anspruch 1, dadurch gekennzeichnet, daß in Formel (I) $R_1$ Wasserstoff, Methyl oder t-Butyl ist.

3. Acetylencarbamid-Derivate wie folgt:

(1) N,N′,N″,N‴-Tetrakis(3-t-butyl-4-hydroxybenzyl)-acetylencarbamid
(2) N,N′,N″,N‴-Tetrakis(3-methyl-5-t-butyl-4-hydroxybenzyl)-acetylencarbamid
(3) N,N′,N″,N‴-Tetrakis(3-ethyl-5-t-butyl-4-hydroxybenzyl)-acetylencarbamid
(4) N,N′,N″,N‴-Tetrakis(3-n-propyl-5-t-butyl-4-hydroxybenzyl)-acetylencarbamid
(5) N,N′,N″,N‴-Tetrakis(3,5-di-t-butyl-4-hydroxybenzyl)-acetylencarbamid
(6) N,N′,N″-Tris(3-t-butyl-4-hydroxybenzyl)-N‴-methylacetylencarbamid
(7) N,N′,N″,-Tris(3-methyl-5-t-butyl-4-hydroxybenzyl)-N‴-methylacetylencarbamid
(8) N,N′,N″,-Tris(3-ethyl-5-t-butyl-4-hydroxybenzyl)-N‴-methylacetylencarbamid
(9) N,N′,N″,-Tris(3-isopropyl-5-t-butyl-4-hydroxybenzyl)-N‴-methylacetylencarbamid
(10) N,N′,N″,-Tris(3,5-di-t-butyl-4-hydroxybenzyl)-N‴-methylacetylencarbamid

4. Verfahren zur Herstellung der Acetylencarbamid-Derivate gemäß Anspruch 1, gekennzeichnet durch die Umsetzung eines Acetylencarbamids der allgemeinen Formel (II)

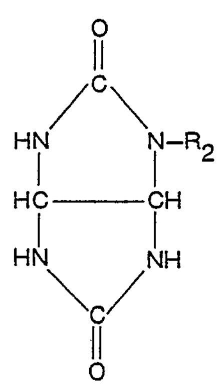

(II)

in der $R_2$ Wasserstoff oder Methyl ist, mit einem p-Hydroxybenzylalkohol-Derivat der allgemeinen Formel (III)

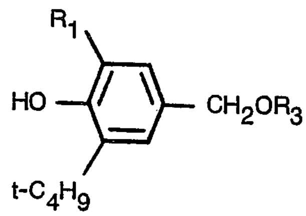

(III)

in der $R_1$ wie in Anspruch 1 definiert und $R_3$ Wasserstoff oder $C_{1-4}$-Alkyl ist, in einem Lösungsmittel in Gegenwart eines basischen katalysators.

5. Verfahren zur Herstellung der Acetylencarbamid-Derivate gemäß Anspruch 1, gekennzeichnet durch die Umsetzung eines Acetylencarbamids der allgemeinen Formel (II)

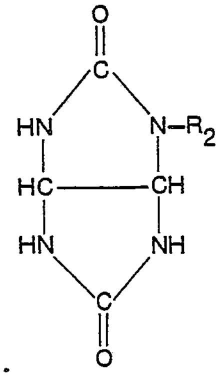

(II)

in der $R_2$ Wasserstoff oder Methyl ist, mit einem Dialkyldithiocarbamat-Derivat der allgemeinen Formel (IV)

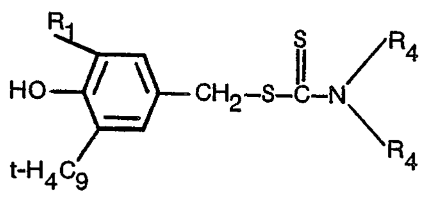

(IV)

in der $R_1$ wie in Anspruch 1 definiert und $R_4$ $C_{1-4}$-Alkyl ist, in einem Lösungsmittel in Gegenwart eines basischen katalysators.

6. Verfahren zur Herstellung der Acetylencarbamid-Derivate gemäß Anspruch 1, gekennzeichnet durch die gleichzeitige Umsetzung eines Acetylencarbamids der allgemeinen Formel (II) gemäß Anspruch 5 und eines Phenols der allgemeinen Formel (V;

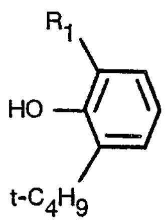

(V)

in der $R_1$ wie in Anspruch 1 definiert ist, in einem Lösungsmittel in Gegenwart eines basischen Katalysators.

7. Verfahren nach Anspruch 4, 5 oder 6, gekennzeichnet durch die Verwendung eines niedrigen Alkohols als Lösungsmittel.

8. Verfahren nach Anspruch 4, 5, 6 oder 7, gekennzeichnet durch die Verwendung eines Alkalimetallalkoxids der Alkalimetallhydroxids als Katalysator.

9. Verfahren nach Anspruch 4, 5, 6, 7 oder 8 gekennzeichnet durch die Umsetzung bei einer Temperatur von 20 bis 200°C.

10. Verfahren nach Anspruch 9, gekennzeichnet durch die Durchführung der Umsetzung unter Rückfluß.

11. Verfahren nach einem der Ansprüche 4 bis 10, gekennzeichnet durch die Verwendung einer Verbindung der Formel (II), in der $R_2$ Wasserstoff und X ein Rest der Formel

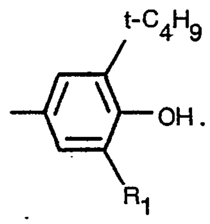

bedeutet.

12. Verfahren nach einem der Ansprüche 4 bis 10, gekennzeichnet durch die Verwendung einer Verbindung der Formel (II), in der $R_2$ Methyl und X Wasserstoff bedeutet.

13. Stabilisator für organische Substanzen, gekennzeichnet durch den Gehalt eines Acetylencarbamid-Derivats gemäß Anspruch 1, 2 oder 3 als Wirkstoff.

14. Stabilisator nach Anspruch 12, gekennzeichnet durch den Gehalt eines Dialkyl-3,3'-Thiodipropionats der allgemeinen Formel (VI)

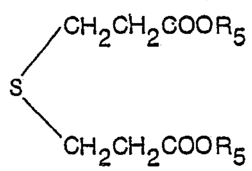

(VI)

in der $R_5$ $C_{12-20}$-Alkyl bedeutet oder eines Pentaerythrit-tetrakis(ß-alkylthiopropionats) der allgemeinen Formel (VII)

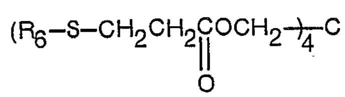

(VII)

in der $R_6$ $C_{4-20}$-Alkyl bedeutet.

15. Stabilisator nach Anspruch 14, gekennzeichnet durch einen Gehalt an Acetylencarbamid-Derivat und Dialkyl-3,3'-thiodipropionat oder Pentaerythrit-tetrakis(ß-alkylthiopropionat) in einem Gewichtsverhältnis von 1:0,5 bis 1:15.

16. Organische Substanz, gekennzeichnet durch die Stabilisierung mit einem Stabilisator gemäß Anspruch 13, 14 oder 15.

17. Organische Substanz nach Anspruch 16 in Form eines Polymers.

18. Organische Substanz nach Anspruch 17, gekennzeichnet durch ein Kunstharz, Kunstgummi oder Naturgummi als Polymer.

19. Organische Substanz nach Anspruch 18, gekennzeichnet durch ein Polyolefin oder ein Acrylnitril-Butadien-Styrol-Copolymer als Kunstharz.

20. Organische Substanz nach Anspruch 19, gekennzeichnet durch Polypropylen als Polyolefin.

21. Organische Substanz nach Anspruch 18, gekennzeichnet durch einen Butadien-Gummi als Kunstgummi.

**Revendications**

1. Dérivé carbamide d'acètylène de formule générale (I)

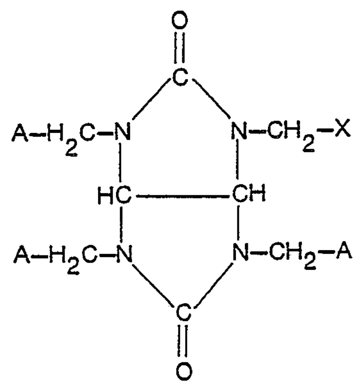

(I)

dans laquelle chaque A représente le groupe

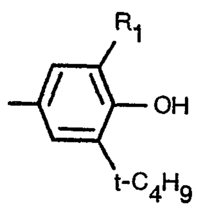

où $R_1$ représente un atome d'hydrogène ou un groupe alkyle ayant de 1 à 4 atomes de carbone et X représente A ou un atome d'hydrogene.

2. Dérivé carbamide d'acétylène selon la revendication 1, dans lequel le substituant $R_1$ représente un atome d'hydrogène, un groupe méthyle ou un groupe t-butyle.

3. Composé choisi parmi les composés (1) à (10):

(1) Carbamide de N,N',N'',N'''-tétrakis(3-t-butyl-4-hydroxybenzyl)acétylèle
(2) Carbamide de N,N',N'',N'''-tétrakis(3-méthyl-5-t-butyl-4-hydroxybenzyl)acétylène
(3) Carbamide de N,N',N'',N'''-tétrakis(3-éthyl-5-t-butyl-4-hydroxybenzyl)acétylène
(4) Carbamide de N,N',N'',N'''-tétrakis(3-n-propyl-5-t-butyl-4-hydroxybenzyl)acétylène
(5) Carbamide de N,N',N'',N'''-tétrakis(3,5-di-t-butyl-4-hydroxybenzyl)acétylène
(6) Carbamide de N,N',N'',-tris(3-t-butyl-4-hydroxybenzyl)-N'''-méthylacétylène.
(7) Carbamide de N,N',N'',-tris(3-méthyl-5-t-butyl-4-hydroxybenzyl)-N'''-méthylacétylène.
(8) Carbamide de N,N',N'',-tris(3-éthyl-5-t-butyl-4-hydroxybenzyl)-N'''-méthylacétylène.
(9) Carbamide de N,N',N'',-tris(3-isopropyl-5-t-butyl-4-hydroxybenzyl)-N'''-méthylacétylène.
(10) Carbamide de N,N',N'',-tris(3,5-di-t-butyl-4-hydroxybenzyl)-N'''-méthylacétylène.

4. Procédé pour la préparation d'un dérivé carbamide d'acétylène selon la revendication 1, qui comprend la réaction d'un un carbamide d'acétylène de formule générale (II):

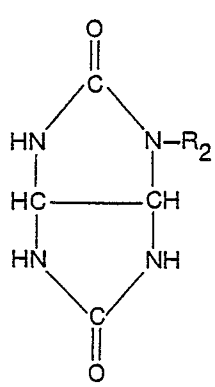

(II)

dans laquelle $R_2$ représente un atome d'hydrogène ou un groupe méthyle, et un dérivé d'alcool p-hydroxybenzylique de formule générale (III):

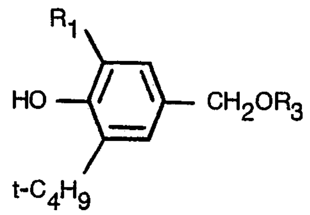

(III)

dans laquelle $R_1$ est comme défini dans la revendication 1, et $R_3$ représente un atome d'hydrogène ou un groupe alkyle ayant de 1 à 4 atomes de carbone, dans un solvant et en présence d'un catalyseur basique.

5. Procédé pour la préparation d'un dérivé carbamide d'acétylène selon la revendication 1, qui comprend la réaction d'un carbamide d'acétylène de formule générale (II):

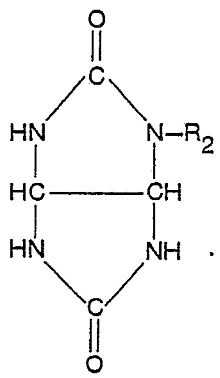

(II)

dans laquelle $R_2$ représente un atome d'hydrogène ou un groupe méthyle, et un dérivé dialkyldithiocarbamate de formule générale (IV):

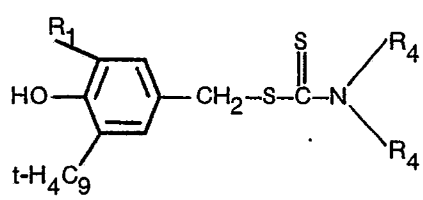

(IV)

dans laquelle $R_1$ est comme défini dans la revendication 1 et $R_4$ représente un groupe alkyle ayant de 1 à 4 atomes de carbone, dans un solvent et en présence d'un catalyseur basique.

6. Procédé pour la préparation d'un dérivé carbamide d'acétylène selon la revendication 1, qui comprend la réaction simultanée d'un carbamide d'acétylène de formule générale (II) tell que définie dans la revendication 5 et d'un phénol de formule générale (V):

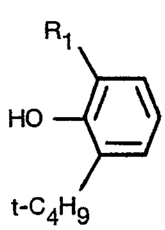

(V)

dans laquelle $R_1$ est comme défini dans la revendication 1, dans un solvant et en présence d'un catalyseur basique.

7. Procédé selon la revendication 4, 5 ou 6, dans lequel ledit solvant est un alcool inférieur.

8. Procédé selon la revendication 4, 5, 6 ou 7, dans lequel ledit catalyseur est un alkoxyde de métal alcalin ou un hydroxyde de métal alcalin.

9. Procédé selon la revendication 4, 5, 6, 7 ou 8, dans lequel le réaction est effectuée à une température de 20 à 200°C.

10. Procédé selon la revendication 9, dans lequel la réaction est effectuée dans des conditions de reflux.

11. Prodédé selon l'une des revendications 4 à 10, dans lequel le substituant $R_2$ représente un atome d'hydrogène et le substituant X représente le groupe

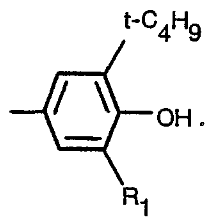

12. Procédé selon l'une des revendications 4 à 10, dans lequel le substituant $R_2$ est un groupe méthyle et le substituant X est un atome d'hydrogène.

13. Stabilisant pour substances organiques qui contient comme ingrédient actif un dérivé carbamide d'acétylène tel que revendiqué dans la revendication 1, 2 ou 3.

14. Stabilisant tel que revendiqué dans la revendication 13, qui contient également un dialkyl-3,3'-thidipropionate de formule générale (VI):

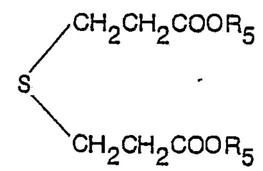

(VI)

dans laquelle $R_5$ représente un groupe alkyle ayant de 12 à 20 atomes de carbone, ou un pentaérythritol-tétrakis(ß-alkylthiopropionate) de formule générale (VII):

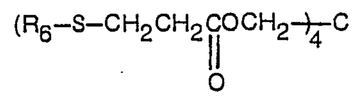

(VII)

dans laquelle $R_6$ représente un groupe alkyle ayant de 4 à 20 atomes de carbone.

15. Stabilisant selon la revendication 14, dans lequel la proportion de dérivé carbamide d'acétylène par rapport au dialkyl-3,3'-thiodipripionate ou au pentaérythritol-tétrakis(ß-alkylthiopropionate) est de 1:0,5 à 1:15 en poids.

16. Substance organique qui a été stabilisée par incorporation dans celle-ci d'un stabilisant tel que revendiqué dans les revendications 13, 14 ou 15.

17. Substance organique selon la revendication 16, qui est un polymère.

18. Substance organique selon la revendication 17, dans laquelle ledit polymère est un résine synthétique, un caoutchouc synthétique ou un caoutchouc naturel.

19. Substance organique selon la revendication 18, dans laquelle ladit résine synthétique est une polyoléfine ou un copolymère acrylonitrile-butadiène-stryène.

20. Substance organique selon la revendication 19, dans laquelle ladite polyoléfine est le polypropylèlne.

21. Substance organique selon la revendication 18, dans laquelle ledit caoutchouc synthétique est un caoutchouc butadiène.